# EUROPÄISCHE PATENTANMELDUNG

(11) **EP 4 706 548 A1**
(43) Veröffentlichungstag der Anmeldung: **11.03.2026**
(21) Anmeldenummer: 25199424.0
(22) Anmeldetag: 01.09.2025
(51) Int. Cl.: A61B 17/00, A61F 9/007

(54) **CHIRURGISCHES SYSTEM FÜR OPHTHALMOLOGISCHE EINGRIFFE**

(30) Priorität: 06.09.2024 DE 102024208484
(71) Anmelder: Geuder AG, 69126 Heidelberg (DE)
(72) Erfinder: EICHHORN, Frank, 74934 Reichartshausen (DE); WOLSCHENDORF, Marc, 64625 Bensheim (DE)
(74) Vertreter: Ullrich & Naumann PartG mbB

(57) **Zusammenfassung**

Ein chirurgisches System für ophthalmologische Eingriffe, umfassend ein Gehäuse (1) mit darin befindlichen Funktionseinheiten sowie mit Bedien- und Anzeigeelementen sowie Anschlüssen für ophthalmologische Geräte, und ggf. umfassend einen Tisch, ein Gestell oder einen Wagen zur Positionierung des Gehäuses (1), ist dadurch gekennzeichnet, dass dem Gehäuse (1) und/oder dem Tisch, Gestell oder Wagen eine vorzugsweise indirekte Beleuchtung (4) als designerisches Element zugeordnet ist, die gleichzeitig einen aktuellen Betriebszustand bzw. eine aktuelle Funktionalität oder ein aktives Programm des Systems anzeigt.

## Beschreibung

Die Erfindung betrifft ein chirurgisches System für ophthalmologische Eingriffe, umfassend ein Gehäuse mit darin befindlichen Funktionseinheiten sowie mit Bedien- und Anzeigeelementen sowie Anschlüssen für ophthalmologische Geräte, und ggf. umfassend einen Tisch, ein Gestell oder einen Wagen zur Positionierung des Gehäuses.

Chirurgische Systeme der hier in Rede stehenden Art sind seit Jahren aus der Praxis bekannt. In Bezug auf die integrierten Funktionseinheiten sowie in Bezug auf die grundsätzliche Funktion sei lediglich beispielhaft auf die DE 10 2017 215 677 B3, DE 10 2015 003 799 A1 und US 2015/0250939 A1 verwiesen. Aus diesen Druckschriften sind, jeweils für sich gesehen, gattungsbildende ophthalmologische Systeme bekannt.

Systeme der hier in Rede stehenden Art umfassen regelmäßig integrierte Lichtquellen, regelmäßig punktuelle Lichtquellen, die einen konkreten Betriebszustand anzeigen können. So verfügen solche Systeme beispielsweise über eine Kontrollleuchte zum Anzeigen des ein- oder ausgeschalteten Zustands. Dort vorgesehene Lichtquellen dienen ausschließlich zum Anzeigen einer singulären, konkreten Situation.

Während Systeme der hier in Rede stehenden Art früher wenig designgeprägt waren, spielt ein modernes Industriedesign eine zunehmende Rolle, ähnlich wie dies bei zahnärztlichen Systemen der Fall ist. Im Vordergrund steht nach wie vor die Funktion bzw. Funktionalität, unmittelbar gefolgt von einem gefälligen Design, welches bei gleichwertigen Vergleichsgeräten kaufentscheidend sein kann.

Ausgehend von der zuvor genannten Situation liegt der Erfindung die Aufgabe zugrunde, designerische Elemente mit Funktionalität zu verknüpfen. Außerdem soll sich der durch das Design verursachte Aufwand durch eine funktionelle Verknüpfung rechtfertigen. Schließlich soll sich das erfindungsgemäße System von wettbewerblichen Produkten unterscheiden.

Voranstehende Aufgabe ist durch die Merkmale des Anspruchs 1 gelöst, wonach das gattungsgemäße chirurgische System für ophthalmologische Eingriffe am Gehäuse und/oder an einem Tisch, Gestell oder Wagen, auf dem das Gehäuse bzw. das Gerät platziert wird, mit einer vorzugsweise indirekten Beleuchtung als designerisches Element ausgestattet ist. Dieses zunächst rein designerische Element wird mit einer funktionalen Aufgabe kombiniert, nämlich dahingehend, dass die Beleuchtung gleichzeitig einen aktuellen Betriebszustand bzw. eine aktuelle Funktionalität oder ein aktives Programm des Systems anzeigt. Die Anzeige dient der Bedienperson, d.h. in erster Linie dem das System anwendenden Arzt sowie den Assistenten.

An dieser Stelle sei angemerkt, dass zunächst einmal die Beleuchtung als designerisches Element definiert ist. Dabei kann es sich beispielsweise um einen Leuchtstreifen, ein beleuchtetes Symbol der Herstellerfirma, ein Symbol der das Gerät betreibenden Klinik oder der das Gerät betreibenden Arztpraxis, etc. handeln. Es ist denkbar, das designerische Element individuell auszugestalten, wodurch eine Art Personalisierung bzw. Individualisierung des Systems möglich ist.

Neben der Aufgabe der Beleuchtung als designerisches Element dient die Beleuchtung dazu, einen aktuellen Betriebszustand dem Nutzer/Anwender bzw. dem begleitenden OP-Personal anzuzeigen. Dabei kann es sich beispielsweise um die Anzeige eines konkret in Aktion befindlichen Programms handeln, beispielsweise in Bezug auf Aspiration oder Irrigation. Auch kann der durch Voreinstellungen am System gewählte Operationsbereich - vorderer Augenabschnitt oder hinterer Augenabschnitt sowie eine bestimmte Phase im OP-Ablauf unterschiedlicher OP-Abschnitte oder unterschiedliche allgemeine Funktionen wie beispielsweise Saugen, Schallen, Spülen, etc. signalisiert werden. Somit wird dem Arzt über die Beleuchtung, vorzugsweise über eine indirekte Beleuchtung, eine Information zur konkreten Funktion vermittelt, ohne dass der Arzt das Gerät direkt beobachten muss. Licht- bzw. Farbreflektionen im Raum reichen zur Informationsvermittlung aus.

Bei der Beleuchtung handelt es sich in vorteilhafter Weise um eine RGB LED-Beleuchtung, so dass ein beliebiger Farbwechsel, insbesondere aber auch ein Farbfluss, möglich ist. So lässt sich beispielsweise durch einen Farbfluss die Strömung in der Irrigations- oder Aspirationsleitung vermitteln.

Entsprechend den voranstehenden Ausführungen emittiert die Beleuchtung entsprechend dem jeweiligen Betriebszustand eine konkrete Farbe oder Farbfolge und/oder ein gleichmäßiges oder pulsierendes oder rotierendes oder fließendes Licht, je nach der "mitzuteilenden" Funktion. Durch die konkrete Wahl der Beleuchtung lassen sich zu vermittelnde Inhalte optimieren und einfachst darstellen.

Die Beleuchtung bzw. Lichtquelle kann in das Gehäuse integriert sein, wobei das emittierte Licht von außen zumindest teilweise sichtbar ist. Auch ist es denkbar, ausgehend von einer punktuellen Lichtquelle Lichtleiter mit definierten Lichtaustritten zu nutzen.

Die Beleuchtung kann an beliebigen Stellen des Gehäuses vorgesehen sein, vorzugsweise in einer seitlichen Gehäusewand, die ansonsten frei von funktionalen Elementen ist. Die Beleuchtung kann sich im Wesentlichen über die gesamte Gehäusewand hinweg erstrecken, entweder durch punktuelle Aneinanderreihung mehrerer Lichtquellen, durch eine linear ausgebildete Lichtquelle oder eine flächige Lichtquelle bzw. entsprechend Licht emittierende Fläche. Lichtleiter mit definierten Lichtaustrittsstellen lassen sich nutzen.

Sofern das System einen Tisch, ein Gestell oder einen Wagen zur Positionierung des Gehäuses nutzt, kann die Beleuchtung - alternativ oder zusätzlich, dem Tisch, Gestell oder Wagen zugeordnet sein. Im Konkreten kann die Beleuchtung, insbesondere die Lichtquelle, einem Rahmen, einer Kante, einem Schenkel, einer Strebe, etc. des Tischs, des Gestells oder des Wagens zugeordnet sein. Beliebige Anbringungen bzw. Integrationen von Licht emittierenden Elementen ist denkbar.

An dieser Stelle sei noch einmal betont, dass es hier nicht um die bloße Vorkehrung einer zusätzlichen Beleuchtung geht, vielmehr um die Vorkehrung einer Beleuchtung als designerisches Element, wobei dieser Beleuchtung gleichzeitig eine Funktion zur Anzeige eines konkreten Betriebszustands hat.

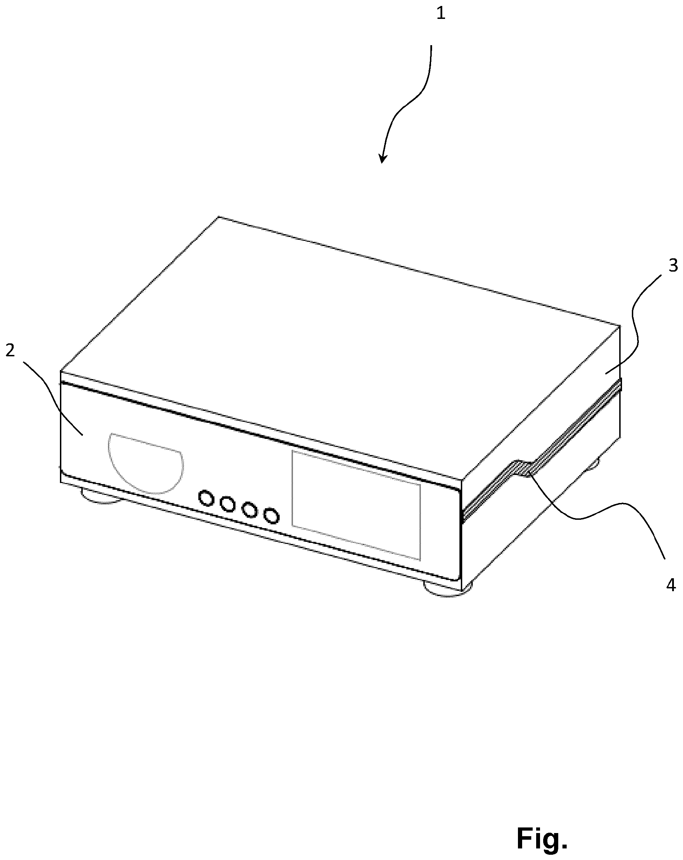

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung eines bevorzugten Ausführungsbeispiels der Erfindung anhand der Zeichnung zu verweisen. In Verbindung mit der Erläuterung eines bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigt die
- einzige Fig.: in einer schematischen Ansicht ein chirurgisches System für ophthalmologische Eingriffe, wobei hier lediglich das Gehäuse, quasi als Blackbox, mit den darin befindlichen Funktionseinheiten gezeigt ist.

Gemäß der Darstellung in der einzigen Figur umfasst das ophthalmologische System ein Gehäuse 1, welches bei dem hier gewählten Ausführungsbeispiel als Tischgerät ausgeführt ist. Es kann ebenso auf einem Gestell, Wagen, etc. angeordnet sein.

Erkennbar ist eine Frontplatte 2 mit Bedien- und Anzeigeelementen, die hier nicht näher erläutert werden. In Bezug auf die Bedien- und Anzeigeelemente sei auf den eingangs genannten Stand der Technik verwiesen.

Die Figur lässt erkennen, dass an bzw. in einer Seitenwand 3 des Gehäuses 1 ein designerisches Element ausgebildet ist, wobei es sich dabei um eine RGB LED-Beleuchtung 4 handelt. Die Beleuchtung 4 bzw. die daraus resultierende Lichtemission ist stilisiert und vermittelt - zunächst - einen designerischen Anspruch des Geräts.

Gleichzeitig dient die Beleuchtung 4 zur Informationsvermittlung dem Operateur und dem assistierenden Personal gegenüber, zeigt nämlich einen aktuellen Betriebszustand bzw. eine aktuelle Funktionalität oder ein aktives Programm des Systems an. Dies kann durch das bloße kontinuierliche Leuchten oder Blinken des Lichts erfolgen. Ebenso lassen sich Informationen über die emittierte Farbe vermitteln.

Es sei an dieser Stelle angemerkt, dass die Beleuchtung 4 und die daraus resultierende Lichtemission beliebig ausgeführt sein kann, beispielsweise linear verlaufend oder flächig. Die Seitenwand bietet sich dazu an, da sie frei von sonstigen funktionalen Bedienelementen ist.

In Bezug auf weitere Merkmale sei zur Vermeidung von Wiederholungen auf den allgemeinen Teil der Beschreibung und die Ansprüche verwiesen.

Hinsichtlich weiterer vorteilhafter Ausgestaltungen der erfindungsgemäßen Lehre wird auf den allgemeinen Teil der Beschreibung sowie auf die beigefügten Ansprüche verwiesen.

Schließlich sei ausdrücklich darauf hingewiesen, dass das voranstehend beschriebenen Ausführungsbeispiel der erfindungsgemäßen Lehre lediglich zur Erörterung der beanspruchten Lehre dient, diese jedoch nicht auf das Ausführungsbeispiel einschränkt.

### Bezugszeichenliste

- 1: Gehäuse
- 2: Frontplatte
- 3: Seitenwand
- 4: Beleuchtung

## Patentansprüche

1. Chirurgisches System für ophthalmologische Eingriffe, umfassend ein Gehäuse (1) mit darin befindlichen Funktionseinheiten sowie mit Bedien- und Anzeigeelementen sowie Anschlüssen für ophthalmologische Geräte, und ggf. umfassend einen Tisch, ein Gestell oder einen Wagen zur Positionierung des Gehäuses (1),
**dadurch gekennzeichnet, dass** dem Gehäuse (1) und/oder dem Tisch, Gestell oder Wagen eine vorzugsweise indirekte Beleuchtung (4) als designerisches Element zugeordnet ist, die gleichzeitig einen aktuellen Betriebszustand bzw. eine aktuelle Funktionalität oder ein aktives Programm des Systems anzeigt.

2. System nach Anspruch 1, **dadurch gekennzeichnet, dass** die Beleuchtung (4) als RGB LED-Beleuchtung ausgeführt ist.

3. System nach Anspruch 1 oder 2, **dadurch gekennzeichnet, dass** die Beleuchtung (4) zumindest teilweise linear und/oder flächig ausgeführt ist.

4. System nach einem der Ansprüche 1 bis 3, **dadurch gekennzeichnet, dass** die Beleuchtung (4) entsprechend dem jeweiligen Betriebszustand eine konkrete Farbe oder Farbfolge und/oder ein gleichmäßiges oder pulsierendes oder rotierendes oder fließendes Licht emittiert.

5. System nach einem der Ansprüche 1 bis 4, **dadurch gekennzeichnet, dass** die Beleuchtung (4) in das Gehäuse (1) integriert und von außen zumindest teilweise sichtbar ist.

6. System nach einem der Ansprüche 1 bis 5, **dadurch gekennzeichnet, dass** die Beleuchtung (4) in eine Gehäusewand, vorzugsweise in eine seitliche Gehäusewand (3), integriert ist.

7. System nach Anspruch 6, **dadurch gekennzeichnet, dass** sich die Beleuchtung im Wesentlichen über die gesamte Gehäusewand hinweg erstreckt.

8. System nacheinem der Ansprüche 1 bis 7 **dadurch gekennzeichnet, dass** die Beleuchtung (4) dem Tisch, Gestell oder Wagen zugeordnet ist.

9. System nach Anspruch 8, **dadurch gekennzeichnet, dass** die Beleuchtung (4) einem Rahmen, einer Kante, einem Schenkel, einer Strebe, etc. des Tischs, Gestells oder Wagens zugeordnet ist.
